# EUROPEAN PATENT APPLICATION

(11) **EP 1 767 193 A2**
(43) Date of publication of application: **28.03.2007**
(21) Application number: 06120628.0
(22) Date of filing: 09.04.2001
(51) Int. Cl.: A61K 9/16, A61K 9/107, A61K 38/13

(54) **Pharmaceutical compositions**

(30) Priority: 10.04.2000 GB 0008785
(62) Divisional of application: 01923719.7
(71) Applicant: Novartis AG, 4002 Basel (CH); Novartis Pharma GmbH, 1230 Vienna (AT)
(72) Inventor: Ambühl, Michael, 4310 Rheinfelden (CH); Haeberlin, Barbara, 4142 Münchenstein (CH); Lückel, Barbara, 79540 Lörrach (DE); Meinzer, Armin, 79426 Buggingen (DE); Lambert, Olivier, 68720 Spechbach-le-Haut (FR); Marchal, Laurent, 54000 Nancy (FR)
(74) Representative: Leon, Susanna Iris

(57) **Abstract**

The present invention provides a pharmaceutical composition in solid form comprising a cyclosporin, a solubilizing component, and a surfactant which is semisolid or solid.

## Description

The present invention relates to novel galenic compositions, in particular novel galenic compositions in which the active ingredient is a poorly water soluble drug e.g. a macrolide, or in particular a cyclic poly-N-methylated undecapeptide, or a cyclosporin. Cyclosporins also include peptolide variants. See e.g. GB patent publications nos. 2 222 770 and 2 257 359 A and equivalents world-wide.

As discussed in the said GB patent publications, the cyclosporins present highly specific difficulties in relation to administration generally and galenic composition in particular, including in particular problems of stability, drug bioavailability, and variability in inter- and intra-patient dose response .

In order to meet these and related difficulties, in GB patent publication no. 2 222 770 and 2 257 359 A, galenic compositions are disclosed comprising a cyclosporin as active ingredient and which take the form of, inter alia, an emulsion, e.g. microemulsion, or emulsion, e.g. microemulsion, pre-concentrate. Such compositions typically comprise 1) a hydrophilic component, 2) a lipophilic component, and 3) a surfactant.

In accordance with the present invention it has now surprisingly been found that particularly suitable galenic compositions with poorly water soluble drugs having particularly interesting bioavailability characteristics and reduced variability in inter- and intra-subject bioavailability parameters, are obtainable using a component which solubilizes the poorly water soluble drug, e.g. a lipophilic component, and a surfactant which is semisolid or solid at room temperature wherein the weight ratio of surfactant to solubilizing component is from about 0.3 - 4, e.g. 1 - 4, to 1. On dilution with an aqueous medium the composition forms an emulsion or microemulsion and/or particulate system.

The present invention provides in one aspect a pharmaceutical composition in solid form comprising
1) a poorly water soluble drug,
2) a solubilzing component, e.g. a lipophilic component
3) a surfactant which is semisolid or solid,
wherein the ratio of surfactant to solubilizing component, e.g. lipophilic component, is from about 0.3 - 4, e.g. 1 - 4, to 1, and
which on dilution with an aqueous medium forms an emulsion or microemulsion and/or a particulate system.

Preferably the composition does not contain any organic hydrophilic component. Under "organic hydrophilic component" is to be understood any hydrophilic component or any hydrophilic co-component as described in the above mentioned British patent application 2 222 770, e.g. no added ethanol, propylene glycol or water, e.g. less than 0.5 % by weight of the composition organic hydrophilic component.

Accordingly, in one aspect the present invention provides a composition as described above which is free, e.g. substantially free, from an organic hydrophilic component.

In one embodiment the present invention further provides a composition which is not an emulsion, e.g. microemulsion, preconcentrate.

The poorly water soluble drug preferably is a lipophilic drug, e.g. a cyclosporin or a macrolide. The term "poorly water soluble", as used herein, is understood to mean a solubility in water at 20°C of less than 1, e.g. 0.01, % weight/volume, e.g. a sparingly soluble to very slightly soluble drug as decribed in Remington: The Science and Practice of Pharmacy, 19th Edition, Ed. A.R. Gennaro, Mack Publishing Company, US, 1995, vol. 1, p 195.

Suitable drugs, e.g. pharmacologically active agents, include e.g. cyclosporins and macrolides.

Cyclosporins to which the present invention applies are any of those having pharmaceutical utility, e.g. as immunosuppressive agents, anti-parasitic agents and agents for the reversal of multi-drug resistance, as known and described in the art, in particular Cyclosporin A (also known as Ciclosporin), Cyclosporin G, [0-(2-hydroxyethyl)-(D)Ser]⁸-Ciclosporin, and [3'-deshydroxy-3'-keto-MeBmt]¹-[Val]²-Ciclosporin. Cyclosporin A is preferred.

In one aspect the present invention provides a composition according to the present invention wherein the cyclosporine is Cyclosporin A.

The term "macrolide" as used herein, refers to a macrocyclic lactone, for example a compound having a 12- membered or larger lactone ring. Of particular interest are the "lactam macrolides", i.e., macrocyclic compounds having a lactam (amide) bond in the macrocycle in addition to a lactone (ester) bond, for example the lactam macrolides produced by microorganisms of the genus Streptomyces such as rapamycin, ascomycin, and FK-506, and their numerous derivatives and analogues. Such lactam macrolides have been shown to have interesting pharmaceutical properties, particularly immunosuppressive and anti-inflammatory properties.

Rapamycin is an immunosuppressive lactam macrolide that is produced by Streptomyces hygroscopicus. The structure of rapamycin is given in Kesseler, H., et al.; 1993; Helv. Chim. Acta; 76: 117. See, e.g., McAlpine, J.B., et al., J. Antibiotics (1991) 44: 688; Schreiber, S.L., et al., J. Am. Chem. Soc. (1991) 113: 7433; US Patent No. 3 929 992. Rapamycin is an extremely potent immunosuppressant and has also been shown to have antitumor and antifungal activity. Its utility as a pharmaceutical, however, is restricted by its very low and variable bioavailability as well as its high toxicity. Moreover, rapamycin is highly insoluble, making it difficult to formulate stable galenic compositions. Numerous derivatives of rapamycin are known. Certain 16-O-substituted rapamycins are disclosed in WO 94/02136, the contents of which are incorporated herein by reference. 40-O-substituted rapamycins are described in, e.g., in US 5 258 389 and WO 94/09010 (O-aryl and O-alkyl rapamycins); WO 92/05179 (carboxylic acid esters), US 5 118 677 (amide esters), US 5 118 678 (carbamates), US 5 100 883 (fluorinated esters), US 5 151 413 (acetals), US 5 120 842 (silyl ethers), WO 93/11130 (methylene rapamycin and derivatives), WO 94/02136 (methoxy derivatives), WO 94/02385 and WO 95/14023 (alkenyl derivatives) all of which are incorporated herein by reference. 32-O-dihydro or substituted rapamycins are described, e.g., in US 5 256 790, incorporated herein by reference.

Rapamycin and its structurally similar analogues and derivatives are termed collectively as "rapamycins".

Ascomycins, of which FK-506 and ascomycin are the best known, comprise another class of lactam macrolides, many of which have potent immunosuppressive and anti-inflammatory activity. FK506 is a lactam macrolide immunosuppressant that is produced by Streptomyces tsukubaensis No 9993. The structure of FK506 is given in the appendix to the Merck Index, 11th ed. (1989) as item A5. Ascomycin is described, e.g., in US patent 3,244,592. Many derivatives of ascomycin and FK-506 have been synthesized, including halogenated derivatives such as 33-epi-chloro-33-desoxy-ascomycin described in EP 427 680. Ascomycin, FK-506 and their structurally similar analogues and derivatives are termed collectively "ascomycins".

The macrolide may, therefore, be rapamycin or an O-substituted derivative in which the hydroxyl group on the cyclohexyl ring of rapamycin is replaced by -OR₁ in which R₁ is hydroxyalkyl, hydroalkoxyalkyl, acylaminoalkyl and aminoalkyl; for example 40-O-(2-hydroxy)ethyl-rapamycin, 40-O-(3-hydroxy)propyl-rapamycin, 40-O-[2-(2-hydroxy)ethoxy]-ethyl-rapamycin and 40-O-(2-acetaminoethyl)-rapamycin.

A preferred compound is 40-0-(2-hydroxy)ethyl rapamycin as disclosed in WO 94/09010.

Examples of compounds of the FK 506 class are those mentioned above. They include for example FK 506, ascomycin and other naturally occurring compounds. They include also synthetic analogues.

A preferred compound of the FK 506 class is disclosed in EP 427 680, e.g. Example 66a also known as 33-epi-chloro-33-desoxy-ascomycin. Other preferred compounds are disclosed in EP 465 426, and in EP 569 337, e.g. the compound of Example 71 in EP 569 337.

In accordance with the present invention, it has surprisingly been found that a cylosporine or macrolide has a high solubility, e.g. a solubility of from about 20 to about 50%, in the solubilizing component, e.g. lipophilic component, of the present invention. The solubilizing component can be one of a large variety of components. A person skilled in the art can choose the appropriate solubilizing component.
The solubilizing, e.g. lipophilic component, is for example:
i) glyceryl mono- or di fatty acid ester, e.g. of C₆-C₁₈, e.g. C₆-C₁₆, e.g. C₈-C₁₀, e.g. C₈, fatty acids, e.g. Sunfat® GDC, or acetylated derivatives thereof, e.g. Myvacet® 9-45 or 9-08, or Imwitor® 308 or 312, and/or
ii) propylene glycol mono- or di- fatty acid ester, e.g. of C₆-C₂₀, e.g. C₈-C₁₂, fatty acids, e.g. Lauroglycol® 90, Sefsol® 218, or Capryol® 90, and/or
iii) fatty acids or alcohols, e.g. C₆-C₂₀, saturated or mono-or di- unsaturated, e.g. oleic acid, oleyl alcohol, linoleic acid, capric acid, caprylic acid, caproic acid, tetradecanol, dodecanol, decanol, and/or
iv) medium chain fatty acid triglycerides, e.g. C₆-C₁₂, e.g. Miglyol® 812, or long chain fatty acid triglycerides, e.g. vegetable oils, and/or
v) mixed mono-, di-, tri-glycerides, e.g. C₆-C₂₀, e.g. C₁₆-C₁₈, e.g. Maisine®, and/or
vi) transesterified ethoxylated vegetable oils, e.g. Labrafil® M2125 CS, and/or
vii) esterified compounds of fatty acid and primary alcohol, e.g. C₈-C₂₀ fatty acids and C₂-C₃ alcohols, e.g. ethyl linoleate, e.g. Nikkol VF-E ®, and/or
viii) glycerol triacetate, e.g. Triacetin, and/or
ix) triethyl citrate, acetyl triethyl citrate, tributyl citrate, acetyl tributyl citrate, and/or
x) hydrocarbons, e.g. squalene, e.g. Squalene®, Squalene Ex®, and/or
xi) ethylene glycol esters, e.g. Monthyle®, and/or
xii) polyglycerol fatty acid esters, e.g. diglyceryl monooleate, e.g. DGMO-C®, DGMO-90®, DGDO®, and/or
xiii) sterols.

For example the solubility of a cyclosporin or a macrolide in Sunfat®GDC-N is about 33%, in Lauroglycol®90 about 40%, in Sefsol®218 about 50%, in oleyl alcohol more than 20%. It is to be appreciated that the solubilizing capacity may depend on the poorly water soluble drug, e.g. pharmacologically active agent, used. In general, for active agents used in dosages of from 0.25 to 100 mg per day, e.g. 0.5 to 10 mg per day, e.g. for macrolides, a solubility of from about 5 to about 10 % in the lipophilic component of the present invention may be desirable. For drugs used in dosages of from 10 to 1000 mg, e.g. 10 to 500 mg, e.g. 50 to 500 mg per day, e.g. for cyclosporins, a solubility of from about 20 to about 50 % in the lipophilic component of the present invention may be desirable.

Accordingly, the present invention provides in one aspect a composition in solid form comprising
1) a cyclosporine or macrolide
2) a solubilizing component, e.g. lipophilic component, wherein component 1) has a solubility of from about 5 to about 50%
3) a surfactant which is semisolid or solid,
wherein the ratio of surfactant to solubilizing component, e.g. lipophilic component, is from about 0.3 - 4, e.g. 1 - 4, to 1 and
which on dilution with an aqueous medium forms an emulsion or microemulsion and/or particulate system.

The solubilizing component, e.g. lipophilic component, may be any one of components i) to xiii) individually or in combination with one, two or more of the other components i) to xiii).

Further details for these solubilizing components, e.g. lipophilic components, are given below.
i) Glyceryl mono- or di- C₆-C₁₈, e.g. C₆-C₁₆, fatty acid ester. Diglycerides suitable for use in the compositions of the invention include both symmetric (i.e. a,a¹-diglycerides) as well as assymetric diglycerides (i.e. a,β-digtycerides) and acetylated derivatives thereof.
   They also include both uniform glycerides (in which the fatty acid constituent is composed primarily of a single fatty acid) as well as mixed glycerides (i.e. in which the fatty acid constituent is composed of various fatty acids) and any acetylated derivatives thereof. The fatty acid constituent may include both saturated and unsaturated fatty acids having a chain length of from C₆-C₁₈, e.g. C₆-C₁₆, e.g. C₈-C₁₀, e.g. C₈. Particularly suitable is caprylic diglyceride which is commercially available, e.g. under the trade name Sunfat® GDC-N, e.g. from Taiyo Kagaku Co., Ltd. Sunfat® GDC-N has an acid value of about 0.3, a diglyceride content of about 78.8%, and a monoester content of about 8.9%.
   Glyceryl mono C₆-C₁₈, e.g. C₆-C₁₄, fatty acid ester may be obtainable by esterification of glycerol with vegetable oil followed by molecular distillation. Monoglycerides suitable for use in the compositions of the invention include both symmetric (i.e. β-monoglycerides) as well as asymmetric monoglycerides (a-monoglycerides) and acetylated derivatives thereof, which may be commercially available, e.g. under the trade name Myvacet®. They also include both uniform glycerides (in which the fatty acid constituent is composed primarily of a single fatty acid) as well as mixed glycerides (i.e. in which the fatty acid constituent is composed of various fatty acids) and any acetylated derivatives thereof. The fatty acid constituent may include both saturated and unsaturated fatty acids having a chain length of from e.g. C₈-C₁₀. Particularly suitable are caprylic or capric acid monoglycerides which are commercially available, e.g. under the trade names Imwitor® 308 or Imwitor® 310, respectively, from e.g. Condea. For example Imwitor® 308 comprises at least 80 % monoglycerides and exhibits the following additional characterising data: free glycerol max 6 %, acid value max. 3, saponification value 245-265, iodine value max. 1, water content max. 1 %. Typically it comprises 1 % free glycerol, 90 % monoglycerides, 7 % diglycerides, 1 % triglycerides (H. Fiedler, "Lexikon der Hilfsstoffe für Pharmazie, Kosmetik und angrenzende Gebiete", Editio Cantor Verlag Aulendorf, Aulendorf, 4th revised and expanded edition (1996), vol 1, page 798).
ii) Propylene glycol mono- or di- C₆-C₂₀ fatty acid ester. The fatty acid constituent may include both saturated and unsaturated fatty acids having a chain length of from e.g. C₈-C₁₂. Particularly suitable are propylene glycol mono ester of caprylic and lauric acid as commercially available, e.g. under the trade names Sefsol® 218, Capryol®90 or Lauroglycol®90, from e.g. Nikko Chemicals Co., Ltd. or Gattefossé. For example Lauroglycol®90 exhibits the following additional characterising data: acid value max. 8, saponification value 200-220, iodine value max. 5, free propylene glycol content max. 5%, monoester content min. 90%; Sefsol® 218 exhibits the following additional characterising data: acid value max. 5, hydroxy value 220-280 (H. Fiedler, loc cit, vol 2, page 906, manufacturer information).
iii) Fatty acids and/or alcohols. Fatty acids may be obtainable by hydrolysis of various animal and vegetable fats or oils, such as olive oil, followed by separation of the liquid acids. The fatty acid/alcohol constituent may include both saturated and mono- or di-unsaturated fatty acids/alcohols having a chain length of from e.g. C₆-C₂₀. Particularly suitable are, e.g. oleic acid, oleyl alcohol, linoleic acid, capric acid, caprylic acid, caproic acid, tetradecanol, dodecanol, or decanol. For example oleyl alcohol is commercially available under the trade mark HD-Eutanol® V from e.g. Henkel KGaA. Oleyl alcohol exhibits the following additional characterising data: acid value max 0.1, hydroxy value of about 210, iodine value of about 95, saponification value max 1, D.²⁰ about 0,849, n_{D}²⁰ 1,462, molecular weight 268, viscosity (20°) about 35 mPa s (manufacturer information). Oleic acid exhibits the following additional characterising data: molecular weight 282,47, D.²⁰ 0,895, n_{D}²⁰ 1,45823, acid value 195-202, iodine value 85-95, viscosity (25°) 26 mPa s (H. Fiedler, loc cit, vol 2, page 1112; "Handbook of Pharmaceutical Excipients", 2nd Edition, Editors A. Wade and P. J. Weller (1994), Joint publication of American Pharmaceutical Association, Washington, USA and The Pharmaceutical Press, London, England, page 325).
iv) As the medium chain fatty acid triglyceride in the lipophilic component a triglyceride of saturated fatty acid having 6 to 12, e.g. 8 to 10, carbon atoms can be used. Suitable medium chain fatty acid triglycerides are those known and commercially available under the trade names Acomed®, Myritol®, Captex®, Neobee®M 5 F, Miglyol®810, Miglyol®812, Miglyol®818, Mazol®, Sefsol®860, Sefsol®870; Miglyol®812 being the most preferred. Miglyol®812 is a fractionated coconut oil comprising caprylic-capric acid triglycerides and having a molecular weight of about 520 daltons. Fatty acid composition = C₆ max. about 3%, C₈ about 50 to 65%, C₁₀ about 30 to 45%, C₁₂ max 5%; acid value about 0.1; saponification value about 330 to 345; iodine value max 1. Miglyol® 812 is available from Condea. Neobee® M 5 F is a fractionated caprylic-capric acid triglyceride available from coconut oil; acid value max. 0.2; saponification value about 335 to 360; iodine value max 0.5, water content max. 0,15%, D.²⁰ 0,930-0,960, n_{D}²⁰ 1,448-1,451 (manufacturer information). Neobee® M 5 F is available from Stepan Europe. In a further alternative aspect the lipophilic component may alternatively comprise e.g. a pharmaceutically acceptable oil, preferably with an unsaturated component such as a vegetable oil.
v) Suitable mixed mono-, di-, tri-glycerides are those known and commercially available under the trade name Maisine® from Gattefossé, They are transesterification products of corn oil and glycerol. Such products are comprised predominantly of linoleic and oleic acid mono-, di- and tri-glycerides together with minor amounts of palmitic and stearic acid mono-, di- and tri-glycerides (corn oil itself being comprised of ca. 56% by weight linoleic acid, 30% oleic acid, ca. 10% palmitic and ca. 3% stearic acid constituents). Physical characteristics are: free glycerol max 10%, monoglycerides ca. 40%, diglycerides ca. 40%, triglycerides ca. 10%, free oleic acid content ca. 1%. Further physical characteristics are: acid value max. 2, iodine value of 85-105, saponification value of 150-175, mineral acid content = 0. The fatty acid content for Maisine® is typically: palmitic acid ca. 11%, stearic acid ca. 2.5%, oleic acid ca. 29%, linoleic acid ca. 56%, others ca. 1.5% (H. Fiedler, loc cit, vol 2, page 958; manufacturer information).
vi) The solubilizing component may alternatively comprise suitable transesterified ethoxylated vegetable oils such as those obtained by reacting various natural vegetable oils (for example, maize oil, kernel oil, almond oil, ground nut oil, olive oil, soybean oil, sunflower oil, safflower oil and palm oil, or mixtures thereof) with polyethylene glycols that have an average molecular weight of from 200 to 800, in the presence of an appropriate catalyst. These procedures are known and an example is described in US Patent 3 288 824. Transesterified ethoxylated corn oil is particularly preferred.
   Transesterified ethoxylated vegetable oils are known and are commercially available under the trade name Labrafil® (H. Fiedler, loc cit, vol 2, page 880). Examples are Labrafil® M 2125 CS (obtained from corn oil and having an acid value of less than about 2, a saponification value of 155 to 175, an HLB value of 3 to 4, and an iodine value of 90 to 110), and Labrafil® M 1944 CS (obtained from kernel oil and having an acid value of about 2, a saponification value of 145 to 175 and an iodine value of 60 to 90). Labrafil® M 2130 CS (which is a transesterification product of a C₁₂₋₁₈ glyceride and polyethylene glycol and which has a melting point of about 35 to 40°C, an acid value of less than about 2, a saponification value of 185 to 200 and an iodine value of less than about 3) may also be used. The preferred transesterified ethoxylated vegetable oil is Labrafil® M 2125 CS which can be obtained, for example, from Gattefossé, Saint-Priest Cedex, France.
vii) As another lipophilic component esterified compounds of fatty acid and primary alcohol may be used. They may include esterified compounds of fatty acid having 8 to 20 carbon atoms and primary alcohol having 2 to 3 carbon atoms, for example, isopropyl myristate, isopropyl palmitate, ethyl linoleate, ethyl oleate, etc., with an esterified compound of linoleic acid and ethanol being particularly preferable.
viii) Glycerol triacetate or (1,2,3)-triacetin. It may be obtainable by esterification of glycerin with acetic anhydride. Glycerol triacetate is commercially available as, e.g. Priacetin® 1580 from Unichema International, or as *Eastman* Triacetin from Eastman, or from Courtaulds Chemicals Ltd. Glycerol triacetate exhibits the following additional characterising data: molecular weight 218,03, D.^{20,3} 1,159-1,163, n_{D}²⁰ 1,430-1,434, water content max. 0.2 %, viscosity (25°) 17.4 mPa s, acid value max. 0.1, saponification value of about 766-774, triacetin content 97% min. (H. Fiedler, loc cit, vol 2, page 1580; Handbook of Pharmaceutical Excipients, loc. cit, page 534, manufacturer information).
ix) Triethyl citrate or acetyl triethyl citrate. They may be obtainable by esterification of citric acid and ethanol or esterification of citric acid and ethanol, followed by acetylation with acetic anhydride, respectively. Triethyl citrate or acetyl triethyl citrate are commercially available, e.g. under the trade names Citroflex® 2 or Citroflex® A-2, or triethyl citrate in a pharmaceutical grade under the name TEC-PG/N, from e.g. Morflex Inc. Particularly suitable is triethyl citrate which has molecular weight of 276,3, a specific gravity of 1,135-1,139, a refractive index of 1,439-1,441, a viscosity (25°) of 35,2 mPa s, assay (anhydrous basis) 99,0-100,5 %, water max. 0,25 % (Fiedler, H. P., loc.cit, , vol 1, page 371; "Handbook of Pharmaceutical Excipients", loc. cit, page 540).
x) Hydrocarbons, e.g. squalene, available from e.g. Nikko Chemicals Co., Ltd.
xi) Ethylene glycol esters, e.g. Monthyle®, available from e.g. Gattefossé.
xii) Polyglycerol fatty acid esters, with e.g. from 2 to 20, e.g. 10 glycerol units. The fatty acid constituent may include both saturated and unsaturated fatty acids having a chain length of from e.g. C₈-C₁₈. Particularly suitable is e.g. diglyceryl monooleate (DGMO), as known and commercially available from e.g. Nikko Chemicals Co., Ltd.
xiii) Sterols and derivatives thereof, for example cholesterols and derivatives thereof, in particular phytosterols, e.g. products comprising sitosterol, campesterol or stigmasterol, and ethylene oxide adducts thereof, for example soya sterols and derivatives thereof, e.g. polyethylene glycol sterols, e.g. polyethylene glycol phytosterols or polyethylene glycol soya sterols. The polyethylene glycols may have e.g. from 10 to 40 [CH₂-CH₂-O] units, e.g. 25 or 30 units. Particularly suitable is polyethylene glycol (30) phytosterol which is commercially available, e.g. under the trade name Nikkol BPS®-30, e.g. from Nikko Chemicals Co., Ltd. Further suitable is polyethylene glycol (25) soya sterol which is commercially available, e.g. under the trade name Generol® 122 E 25, e.g. from Henkel (H. Fiedler, loc cit, vol. 1, p. 680).

Although any pharmaceutically acceptable components selected from the group specified above may be used in the composition of the invention, certain components are preferred. These include oleyl alcohol, Lauroglycol®90, Sefsol® 218, Capryol®90 or Sunfat® GDC-N.

Accordingly, the present invention provides in one aspect a composition according to the present invention, wherein the solubilizing component, e.g. lipophilic component, is selected from the group consisting of
(i) glyceryl di C₆-C₁₆ fatty acid ester,
(ii) propylene glycol mono C₆-C₁₂ fatty acid ester,
(iii) fatty acids and alcohols.

In the pharmaceutical composition of the present invention, in a further alternative aspect the constitutional ratio of the solubilizing component, e.g. lipophilic component, : cyclosporin may be from about 15 to 1 : 1 and preferably from about 10 to 1.5 : 1, on the basis of weight.

The term "semisolid or solid", as used herein, is understood to mean a surfactant having a melting point of e.g. above 30°C to about 40°C (semisolid) or above 40°C (solid), respectively .

Examples of suitable surfactants for use in this invention are:
i) Reaction products of a natural or hydrogenated castor oil and ethylene oxide. The natural or hydrogenated castor oil may be reacted with ethylene oxide in a molar ratio of from about 1:35 to about 1:60, with optional removal of the polyethyleneglycol component from the products. Various such surfactants are commercially available. The polyethyleneglycol-hydrogenated castor oils available under the trade name Cremophor® are especially suitable. Particularly suitable are Cremophor® RH 40, which has a saponification value of about 50 to 60, an acid value less than about 1, a water content (Fischer) less than about 2%, an n_{D}⁶⁰ of about 1.453 to 1.457 and an HLB of about 14 to 16; and Cremophor® RH 60, which has a saponification value of about 40 to 50, an acid value less than about 1, an iodine value of less than about 1, a water content (Fischer) of about 4.5 to 5.5%, an n_{D}⁶⁰ of about 1.453 to 1.457 and an HLB of about 15 to 17. An especially preferred product of this class is Cremophor® RH40.
   Similar or identical products which may also be used are available under the trade names Nikkol® (e.g. Nikkol® HCO-40 and HCO-60), Mapeg® (e.g. Mapeg® CO-40h), Incrocas® (e.g. Incrocas® 40), Tagat® (for example polyoxyethylene-glycerol-fatty acid esters e.g. Tagat® RH 40) and Simulsol OL-50 (PEG-40 castor oil, having a saponification value of about 55 to 65, an acid value of max. 2, an iodine value of 25 to 35, a water content of max. 8%, and an HLB of about 13, available from Seppic). These surfactants are further described in Fiedler loc. cit..
ii) Polyoxyethylene fatty acid esters, for example polyoxyethylene stearic acid esters of the type known and commercially available under the trade name Myrj® from ICI (Fiedler, loc. cit., 2, p. 1042). An especially preferred product of this class is Myrj® 52 having a D²⁵ of about 1.1., a melting point of about 40 to 44°C, an HLB value of about 16.9., an acid value of about 0 to 1 and a saponification no. of about 25 to 35.
iii) Polyoxyethylene-polyoxypropylene co-polymers and block co-polymers, poloxamers, for example of the type known and commercially available under the trade names Pluronic®, Emkalyx® (Fiedler, loc. cit., 2, p. 1203). An especially preferred product of this class is Pluronic® F68 (poloxamer 188), having a melting point of about 52°C and a molecular weight of about 6800 to 8975.
iv) Polyoxyethylene mono esters of a saturated C₁₀ to C₂₂, e.g. C₁₈ substituted e.g. hydroxy fatty acid; e.g. 12 hydroxy stearic acid PEG ester, e.g. of PEG about e.g. 600-900 e.g. 660 daltons MW, e.g. Solutol® HS 15 from BASF, Ludwigshafen, Germany.
v) Polyoxyethylene alkyl ethers, e.g. polyoxyethylene glycol ethers of C₁₂ to C₁₈ alcohols, e.g. Polyoxyl 2-, 10- or 20-cetyl ether or Polyoxyl 23-lauryl ether, or polyoxyl 20-oleyl ether, or Polyoxyl 2-, 10-, 20- or 100-stearyl ether, as known and commercially available e.g. under the trade mark Brij® from ICI. An especially preferred product of this class is e.g. Brij® 35 (Polyoxyl 23 lauryl ether) or Brij® 98 (Polyoxyl 20 oleyl ether) (Fiedler, loc. cit., 1, pp. 259; Handbook of Pharmaceutical Excipients, loc. cit., page 367).
   Similar products which may also be used are polyoxyethylene-polyoxypropylene-alkyl ethers, e.g. polyoxyethylene-polyoxypropylene- ethers of C₁₂ to C₁₈ alcohols, e.g. polyoxyethylen-20-polyoxypropylene-4-cetylether which is known and commercially available under the trade mark Nikkol PBC® 34, from e.g. Nikko Chemicals Co., Ltd. (Fiedler, loc. cit., vol. 2, pp. 1239). Polyoxypropylene fatty acid ethers, e.g. Acconon® E may also be used.
vi) Sodium alkyl sulfates and sulfonates, and sodium alkyl aryl sulfonates, e.g. sodium lauryl sulfate, which is also known as sodium dodecyl sulfate and which is commercially available, e.g. under the trade name Texapon K12® from Henkel KGaA.
vii) Water soluble tocopheryl polyethylene glycol succinic acid esters (TPGS), e.g. with a polymerisation number ca 1000, e.g. available from Eastman Fine Chemicals Kingsport, Texas, USA.
viii) Alkylene polyol ether or ester. It may be suitably a C₃₋₅alkylene triol, in particular glycerol, ether or ester. Suitable C₃₋₅alkylene triol ether or ester include mixed ethers or esters, i.e. components including other ether or ester ingredients, for example transesterification products of C₃₋₅alkylene triol esters with other mono-, di- or poly-ols. Particularly suitable alkylene polyol ether or ester are mixed C₃₋₅alkylene triol/poly-(C₂-₄alkylene) glycol fatty acid esters, especially mixed glycerol/polyethylene- or polypropylene-glycol fatty acid esters.
   Especially suitable alkylene polyol ether or ester for use in accordance with the present invention include products obtainable by transesterification of glycerides, e.g. triglycerides, with poly-(C₂₋₄alkylene) glycols,e.g. poly-ethylene glycols and, optionally, glycerol.
   Such transesterification products are generally obtained by alcoholysis of glycerides, e.g. triglycerides, in the presence of a poly-(C-₂₋₄alkylene) glycol, e.g. polyethylene glycol and, optionally, glycerol (i.e. to effect transesterification from the glyceride to the poly-alkylene glycol/glycerol component, i.e. via poly-alkylene glycolysis/glycerolysis).
   In general such reaction is effected by reaction of the indicated components (glyceride, polyalkylene glycol and, optionally, glycerol) at elevated temperature under an inert atmosphere with continuous agitation.

Preferred glycerides are fatty acid triglycerides, e.g. (C₁₀₋₂₂fatty acid) triglycerides, including natural and hydrogenated oils, in particular vegetable oils. Suitable vegetable oils include, for example, olive, almond, peanut, coconut, palm, soybean and wheat germ oils and, in particular, natural or hydrogenated oils rich in (C₁₂₋₁₈fatty acid) ester residues.
Preferred polyalkylene glycol materials are polyethylene glycols, in particular polyethylene glycols having a molecular weight of from ca. 500 to ca. 4,000, e.g. from ca. 1,000 to ca. 2,000.
Suitable alkylene polyol ether or ester thus comprise mixtures of C₃₋₅alkylene triol esters, e.g. mono-, di- and tri-esters in variable relative amount, and poly (C₂₋₄alkylene) glycol mono- and di-esters, together with minor amounts of free C₃₋₅alkylene triol and free poly-(C₂₋₅alkylene) glycol. As hereinabove set forth, the preferred alkylene triol moiety is glyceryl; preferred polyalkylene glycol moieties wil be polyethylene glycol, in particular having a molecular weight of from ca. 500 to ca. 4,000; and preferred fatty acid moieties will be C₁₀₋₂₂fatty acid ester residues, in particular saturated C₁₀₋₂₂fatty acid ester residues.
Particularly suitable alkylene polyol ether or ester may thus alternatively be defined as:
transesterification products of a natural or hydrogenated vegetable oil and a polyethylene glycol and, optionally, glycerol; or compositions comprising or consisting of glyceryl mono-, di- and tri-C₁₀₋₂₂fatty acid esters and polyethylene glycol mono- and di-C₁₀₋₂₂fatty esters (optionally together with, e.g. minor amounts of free glycerol and free polyethylene glycol).
Preferred vegetable oils, polyethylene glycols or polyethylene glycol moieties and fatty acid moieties in relation to the above definitions are as hereinbefore set forth.
Particularly suitable alkylene polyol ether or ester as described above for use in the present invention are those known and commercially available under the trade name Gelucire® from e.g. Gattefossé, in particular the products
a) Gelucire® 33/01, which has an m.p. = ca. 33-38°C
   and a saponification value = ca. 240/260;
b) Gelucire® 35/10, m.p. = ca. 29-34°C, saponification v. = ca. 120-140;
c) Gelucire® 37/02, m.p. = ca. 34-40°C, saponification v. = ca. 200-220;
d) Gelucire® 42/12, m.p. = ca. 41-46°C, saponification v. = ca. 95-115;
e) Gelucire® 44/14, m.p. = ca. 42-46°C, saponification v. = ca. 75-95;
f) Gelucire® 46/07, m.p. = ca. 47-52°C, saponification v. = ca. 125-145;
g) Gelucire® 48/09, m.p. = ca. 47-52°C, saponification v. = ca. 105-125;
h) Gelucire® 50/02, m.p. = ca. 48-52°C, saponification v. = ca. 180-200;
i) Gelucire® 50/13, m.p. = ca. 46-51°C, saponification v. = ca. 65-85;
j) Gelucire® 53/10, m.p. = ca. 48-53°C, saponification v. = ca. 95-115;
k) Gelucire® 62/05, m.p. = ca. 60-65°C, saponification v. = ca. 70-90.

Products (a) to (j) above all have an acid value of max. 2. Product (k) has an acid value max. 5. Products (b), (c) and (f) to (j) above all have an iodine value of max. 3. Product (a) has an iodine value of max. 8. Products (d) and (e) have an iodine value of max. 5 or 2. Product (k) has an iodine value of max. 10.
Alkylene polyol ether or ester having an iodine value of max. 2 will generally be preferred. As will be appreciated, mixtures of alkylene polyol ether or ester as defined may also be employed in the compositions of the invention.

Gelucire® products are inert semi-solid waxy materials with amphiphilic character. They are identified by their melting point and their HLB value. Most Gelucire® grades are saturated polyglycolised glycerides obtainable by polyglycolysis of natural hydrogenated vegetable oils with polyethylene glycols. They are composed of a mixture of mono-, di-and tri-glycerides and mono- and di-fatty acid esters of polyethylene glycol. Particularly suitable is Gelucire® 44/14 which has a nominal melting point of 44°C and an HLB of 14. It is derived from the reation of hydrogenated palm kernel and/or hydrogenated palm oils with polyethylene glycol 1500. It consists of approximately 20% mono-, di- and triglycerides, 72 % mono- and di- fatty acid esters of polyethylene glycol 1500 and 8% of free polyethylene glycol 1500. The fatty acid distribution for Gelucire® 44/14 is as follows: 4-10 C₈, 3-9 C₁₀, 40-50 C₁₂, 14-24 C₁₄, 4-14 C₁₆, 5-15 C₁₈. Gelucire® 44/14 exhibits the following additional characterising data: acid value of max. 2, iodine value of max. 2, saponification value of 79-93, hydroxyl value of 36-56, peroxide value of max. 6, alkalines impurities max. 80, water content max. 0.50, free glycerol content max. 3, monoglycerides content 3.0-8.0. (H. Fiedler, loc cit, vol 1, page 676; manufacturer information).
ix) Polyethylene glycol glyceryl fatty acid ester. The fatty acid ester may include mono and/or di and/or tri fatty acid ester. The fatty acid constituent may include both saturated and unsaturated fatty acids having a chain length of from e.g. C₁₂-C₁₈. The polyethylene glycols may have e.g. from 10 to 40 [CH₂-CH₂-O] units, e.g. 15 or 30 units. Particularly suitable is polyethylene glycol (15) glyceryl monostearat which is commercially available, e.g. under the trade name TGMS®-15, e.g. from Nikko Chemicals Co., Ltd.
x) Sugar fatty acid esters of e.g. C₁₂-C₁₈ fatty acids, e.g. sucrose monolaurate, e.g. Ryoto L-1695® as known and commercially available from e.g. Mitsubishi-Kasei Food Corp., Tokyo, Japan.
xi) PEG sterol ethers having, e.g. from 5 to 35 [CH₂-CH₂-O] units, e.g. 20 to 30 units., e.g. Solulan® C24, as known and commercially available from e.g. Amerchol.
xii) Salts of, e.g. C₆-C₁₈, fatty acids, -fatty acid sulfates and sulfonates, as known and commercially available from e.g. Fluka.
xiii) Salts of, e.g. C₆-C₁₈, acylated amino acids, e.g. sodium lauroyl sarcosinate, as known and commercially available from e.g. Fluka.
xiv) Medium or long-chain alkyl, e.g. C₆-C₁₈, ammonium salts, e.g. cetyl trimethyl ammonium bromide, as known and commercially available from e.g. E. Merck AG.

It is to be appreciated that surfactants may be complex mixtures containing side products or unreacted starting products involved in the preparation thereof, e.g. surfactants made by polyoxyethylation may contain another side product, e.g. polyethylene glycol.

A surfactant having a hydrophilic-lipophilic balance (HLB) value of 8 to 17 is preferred. The surfactant selected preferably has a hydrophilic-lipophilic balance (HLB) of at least 10, for example Cremophor. The HLB value is preferably the mean HLB value.

In one aspect the present invention provides a composition according to the present invention wherein the surfactant is a reaction product of natural or hydrogenated vegetable oil and ethylene oxide, or sodium lauryl sulfate, preferably sodium lauryl sulfate.

In the pharmaceutical composition of the present invention, in a further alternative aspect the constitutional ratio of the surfactant : drug, e.g. cyclosporin, may be from about 0.6 - 80, e.g. 1 - 80, : 1 and preferably from about 1.5 to 25 : 1, on the basis of weight.

Preferably the composition on dilution with an aqueous medium, for example water, for example on dilution of 1:1 to 1:300, e.g. 1:1 to 1:70, e.g. 1:10 to 1:70, e.g. 1:10, or in the gastric juices after oral application, spontaneously forms an o/w (oil-in-water) emulsion, e.g. microemulsion.

A microemulsion is thermodynamically stable and contains dispersed particles of a mean size less than about 200 nm. Generally microemulsions comprise droplets or particles having a mean diameter of less than about 150 nm; typically less than 100 nm, generally greater than 10 nm, and stable over periods in excess of 24 hours. A "microemulsion" may be a non-opaque or substantially non-opaque, alternatively it may be a translucent colloidal dispersion that is formed spontaneously or substantially spontaneously when its components are brought into contact. Further characteristics can be found in the above mentioned British patent application 2 222 770, the disclosure of which is incorporated herein by reference.

In one aspect the present invention provides a composition according to the present invention, the relative proportion of the poorly water soluble drug, e.g. cyclosporine or macrolide, the solubilizing component, and the surfactant in said composition being such that upon dilution with water, for example in a ratio of 1:1 to 1:300, e.g. 1:1 to 1:70, e.g. 1:10 to 1:70, e.g. 1:10, an oil-in-water microemulsion having particles of a mean size of less than 200 nm, is spontaneously formed.

Preferably, after dilution of the composition in an aqueous medium, the relative proportion of the solubilizing component and the surfactant lie within the "microemulsion" region on a standard three way plot. The compositions thus obtained are of high stability that are capable, on addition to an aqueous medium, of providing microemulsions having a mean particle size of < 200 nm.

Standard three way plots, e.g. phase diagrams, can be generated in a conventional manner as described in e.g. GB patent publication no. 2 222 770 or WO 96/13273.

In a further aspect of the present invention, after dilution with an aqueous medium a paticulate system, e.g. of solid particles of the drug, e.g. of a size of from 50 nm to 2000 nm, is formed, e.g. in addition to the emulsion or microemulsion as described above.

The drug may be present in an amount by weight of up to about 35 % by weight of the composition. The drug is preferably present in an amount of 1 to 25 % by weight of the composition, for example about 2 to 20 %.

In one aspect the present invention provides a composition according to the present invention comprising the cyclosporine or macrolide in an amount of 1 to 35% by weight of the composition.

In a further alternative aspect the solubilizing, e.g. lipophilic component, may comprise 10 to 75 %, e.g. 10 to 50 %, by weight of the total weight of the composition, e.g. 15 to 45 %; preferably 20 to 40 % by weight of the composition.

In a further alternative aspect the surfactant may comprise 20 to 90 % by weight of the total weight of the composition, preferably 30 to 80 % by weight, more preferably 40 to 70 % by weight of the composition.

In one aspect the present invention provides a composition according to the present invention comprising the solubilizing, e.g. lipophilic component, in an amount of 10 to 75 %, e.g. 10 to 50 %, and the surfactant in an amount of 20 to 90 % by weight of the total weight of the composition.

Another substance which may be present is a carrier, e.g. a solid carrier. Suitable carriers for use according to the present invention, e.g. to obtain compositions in solid form, e.g. powder form, are, e.g. polymers, e.g. water soluble polymers, e.g. polyethylene glycol or polyvinylpyrrolidone, maltodextrin, e.g. Glucidex®, gummi arabicum, or gelatine; or water insoluble polymers, e.g. microcrystalline cellulose and derivatives thereof, or colloidal silica, e.g. Aerosil®; or lactose; or dibasic anhydrous calcium phosphate, e.g. Fujicalin®.

In one aspect the present invention provides a composition according to the present invention wherein a carrier, e.g. a polymer, e.g. maltodextrin, gummi arabicum, or gelatine, or lactose is additionally present.

In the pharmaceutical composition according to the present invention, in a further alternative aspect the ratio of drug and solubilizing component : carrier is preferably in the range of 1 : 0.5 - 5, e.g. 1 : 1 - 5, more preferably 1 : 1 - 2 on the basis of weight.

In a further alternative aspect the present invention provides a composition according to the present invention wherein the solubilizing component and the drug are encapsulated in a polymeric matrix, e.g. according to a process comprising the following steps: (i) dissolving the drug in the solubilizing, e.g lipophilic, component; (ii) mixing the solution obtained by step (i) with a solution of a polymer in a suitable, e.g. organic, solvent; (iii) delivering the monophasic system containing the polymer, the solubilizing, e.g. lipophilic, component and the drug to a mixer together with e.g. a buffered gelatin solution to form e.g. an o/w emulsion; (iv) hardening the microparticles by solvent evaporation, washing for excipients removal and receiving the microparticles. In order to e.g. increase flowability of the final microparticle powder, the obtained microparticles may be further worked up by adding an aqueous solution of a carrier, e.g. lactose, and lyophilization of the resulting suspension to obtain a flowable powder.

Accordingly, in one aspect the present invention provides a composition of the invention
wherein the drug dissolved in the solubilizing component is encapsulated in a polymeric matrix.

According to the present invention the polymeric matrix may comprise e.g. a water soluble polymer, e.g. polyethylene glycol or polyvinylpyrrolidone, or a water insoluble polymer, e.g. d,l-poly(lactide-co-glycolide), especially d,l-poly(lactide-co-glycolide)/glucose. In a further alternative aspect of the present invention, e.g. to control drug release kinetics, mixtures of polymers, e.g. of a water insoluble polymer, e.g. d,l-poly(lactide-co-glycolide), e.g. d,l-poly(lactide-co-glycolide)/glucose, and a water soluble polymer, e.g. polyethylene glycol, or polyvinylpyrrolidone, or a polymer of dimethylaminoethylmethacrylates and methacrylic acid esters, e.g. Eudragit® E, may be used.

The polymer used to encapsulate the drug together with the solubilizing component may be present in an amount of from 20 to 80 %, e.g. 40 to 50 %, by weight of the total weight of the microparticles comprising e.g. drug, solubilizing component and polymer.

In a further alternative aspect the invention also provides a process for the production of a pharmaceutical composition as defined above, e.g. in solid form, e.g. powder form, which process may comprise (i) dissolving the drug in the solubilizing component; (ii) encapsulating the solution obtained by step (i) in a polymeric matrix; (iii) spray drying or freeze drying the microparticles obtained by step (ii), optionally together with a suitable carrier, to obtain e.g. a powder; (iv) admixing the composition, e.g. powder, obtained by step (iii) with the surfactant.

In a further alternative aspect the present invention provides a composition of the invention which is in freeze-dried form.

Typically, when the compositions of the invention are formulated according to the process described above, the weight ratio of the sum of (i) drug, e.g. cyclosporin or macrolide, (ii) solubilizing component, and (iii) polymer: (iv) carrier may be from (i, ii, and iii) 1 : (iv) 0.1 - 2.

The surfactant may be present in an amount of from 5 to 60 %, e.g. 10 to 55 %, e.g. 50 %, by weight of the total weight of the composition comprising e.g. drug, solubilizing component, polymer, carrier and surfactant.

In yet a further alternative aspect the invention also provides a process for the production of a pharmaceutical composition as defined above, e.g. in solid form, e.g. powder form, which process may comprise (i) dissolving the surfactant in an aqueous solution, (ii) dissolving the drug in the solubilizing component, e.g. lipophilic component, (iii) mixing the aqueous solution of the surfactant with the drug solubilized in the lipophilic component, and (iv) spray-drying the mixture together with a suitable carrier in a conventional manner.

Accordingly, in one aspect the present invention provides a composition of the invention which is in spray-dried form.

Typically, when the compositions of the invention are formulated according to the process described above, the weight ratio of the sum of (i) drug, e.g. cyclosporin or macrolide, (ii) solubilizing component, and (iii) surfactant : (iv) carrier may be from (I, ii, and iii) 1 - 3 : (iv) 0.25 - 4.

The compositions, e.g. those in the examples hereinafter, show good stability characteristics as indicated by standard stability trials, for example having a shelf life stability of up to one, two or three years, and even longer. The compositions of this invention may produce stable emulsions or microemulsions and/or particulate systems, e.g. for up to one day or longer, e.g. one day.

The pharmaceutical composition may also include further additives or ingredients, for example antioxidants, such as ascorbyl palmitate, butyl hydroxy anisole (BHA), butyl hydroxy toluene (BHT) and tocopherols, and/or preserving agents. In a further alternative aspect these additives or ingredients may comprise about 0.05 to 1 % by weight of the total weight of the composition. The pharmaceutical composition may also include sweetening or flavoring agents in an amount of up to about 2.5 or 5% by weight based on the total weight of the composition. Preferably the antioxidant is α-tocopherol (vitamin E).

Details of excipients of the invention are described in e.g. Fiedler, H. P., loc cit; "Handbook of Pharmaceutical Excipients", loc cit; or may be obtained from the relevant manufacturers, the contents of which are hereby incorporated by reference.

Any carbon chain not otherwise specified herein conveniently contains 1 to 18 carbon atoms, e.g. 10 to 18 carbon atoms, when a terminal group or 2 or 3 carbon atoms when a polymer moiety.

It will be appreciated that the present invention encompasses
a) in respect of component (2) any of components i) to xiii) individually or in combination with one, two or more of the other components i) to xiii),
b) in respect of component (3) any of the surfactants specified above, e.g. surfactants i) to xiv), individually or in combination.

When required, the composition of the invention may be compounded into unit dosage form, for example filling the composition into gelatine capsules, e.g. hard gelatine capsules.

Alternatively, the powder composition may be compressed into tablets in a conventional manner.

The composition of the invention may be combined with water or an aqueous solvent medium such that an emulsion, e.g. microemulsion, and/or a particulate system, is obtained. The emulsion, e.g. microemulsion, and/or particulate system, may be administered enterally, e.g orally, e.g. as a capsule, e.g. soft gelatine capsule, or parenterally, e.g. as an infusion concentrate. Oral administration is preferred.

The compositions of the invention in solid form, e.g. powder form, e.g. spray-dried or freeze-dried form, are particularly suitable for the formulation of solid oral dosage forms, e.g. hard gelatine capsules or tablets.

It has also been found that stable compositions containing macrolides may be obtained by formulating the macrolide in an acidic environment. Compositions are understood herein to be stable when the macrolide drug substance remains substantially intact after a period of days or weeks at room temperature (25°C).

The acid may be lipid soluble and/or ethanol soluble. The acid may be for example a fatty acid, e.g. oleic acid. The acid may be a carboxylic acid, for example a mono-, di- or tricarboxylic acid, and preferably a mono- or dicarboxylic acid. The acid may comprise one or more hydrophilic groups, e.g. hydroxy groups, and preferably one or two hydrophilic groups. Suitable acids for use in this invention include malonic acid, fumaric acid, maleic acid, D-malic acid, L-malic acid, citric acid, ascorbic acid, succinic acid, oxalic acid, benzoic acid or lactic acid or an acid with a similar pKa, e.g. 2-7. Preferred acids include malonic acid, oxalic acid, citric acid and lactic acid. Malonic acid is more preferred.

The preferred amount of acid may be determined by routine experimentation. The ratio by weight of macrolide to acid in the compositions of this invention may be up to 20:1, for example from 1:5 to 5:1, e.g. 1:1. In a further alternative aspect the acid may be present in an amount of between 0.05% and 5% by weight of the composition.

In a further alternative aspect the macrolide may be present in an amount of 1 to 15 % by weight of the composition.

The macrolide may, for example, be formulated into a composition according to the present invention as defined above, and combined with an amount of acid. The acid-stabilised composition may be administered enterally, e.g orally, e.g. as a capsule or drink solution, or parenterally, e.g. as an infusion concentrate. Oral administration is preferred.

The pharmaceutical compositions of the invention exhibit especially advantageous properties when administered orally; for example in terms of consistency and high level of bioavailability obtained in standard bioavailability trials. These trials are performed in animals e.g. rats or dogs or healthy volunteers using HPLC or a specific or nonspecific monoclonal kit to determine the level of the drug substance, e.g. cyclosporin macrolide in the blood. For example, the composition of Example 1 administered p.o. to dogs may give surprisingly high Cₘₐₓ values as detected by ELISA using a specific monoclonal antibody.

In one aspect the present invention provides a method of orally administering a pharmaceutical composition, said method comprising orally administering to a patient in need of cyclosporin or macrolide therapy a composition according to the present invention.

Pharmacokinetic parameters, for example absorption and blood levels, also become surprisingly more predictable and problems in administration with erratic absorption may be eliminated or reduced. Additionally the pharmaceutical compositions are effective with biosurfactants or tenside materials, for example bile salts, being present in the gastro-intestinal tract. That is, the pharmaceutical compositions of the present invention are fully dispersible in aqueous systems comprising such natural tensides and thus capable of providing emulsion or microemulsion systems and/or particulate systems in situ which are stable. The function of the pharmaceutical compositions upon oral administration remain substantially independent of and/or unimpaired by the relative presence or absence of bile salts at any particular time or for any given individual.

The compositions of this invention reduce variability in inter- and intra-patient dose response.

In one aspect the present invention provides a method of reducing the variability of bioavailability levels of a cyclosporin or macrolide for patients during cyclosporin or macrolide therapy, said method comprising orally administering an oral pharmaceutical composition according to the present invention.

The utility of all the pharmaceutical compositions of the present invention may be observed in standard clinical tests in, for example, known indications of drug dosages giving equivalent blood levels of drug; for example using dosages in the range of 2.5 mg to 1000 mg of drug per day for a 75 kilogram mammal, e.g. adult and in standard animal models. The increased bioavailability of the drug provided by the compositions may be observed in standard animal tests and in clinical trials, e.g. as described above.

The optimal dosage of drug to be administered to a particular patient may be considered carefully as individual response to and metabolism of the drug, e.g. cyclosporin or macrolide, may vary, e.g. by monitoring the blood serum levels of the drug by radioimmunoassay, monoclonal antibody assay, or other appropriate conventional means. Dosages of the e.g.macrolide will generally range from 1 to 1000 mg per day, e.g. 2.5 mg to 1000 mg per day for a 75 kilogram adult, preferably 25 mg to 500 mg, with the optimal dosage being approximately 50 to 100 mg per day. Satisfactory results are obtained by administering about 75 mg per day for example in the form of two capsules, one containing 50 mg and one containing 25 mg; or three capsules each containing 25 mg. Cyclosporin dosages may be 25 to 1000 mg per day (preferably 50 mg to 500 mg) and the FK 506 dosage may be 1 mg to 1000 mg, e.g. 2.5 to 1000 mg, per day (preferably 10 mg to 250 mg). A daily dosage of between 0.01 and 5 mg/kg body weight/day, e.g. 0.5 and 5 mg/kg body weight/day, is indicated for administration of 40-0-(2-hydroxy)ethyl rapamycin.

The pharmaceutical compositions are preferably compounded in unit dosage form, for example by filling them into orally administrable capsule shells. The capsule shells may be soft or hard gelatine capsule shells, preferably hard gelatine capsule shells. Where the pharmaceutical composition is in unit dosage form, each unit dosage will suitably contain between 10 and 100 mg of the drug, more preferably between 10 and 50 mg; for example 15, 20, 25, or 50 mg. Such unit dosage forms are suitable for administration 1 to 5 times daily depending upon the particular purpose of therapy, the phase of therapy and the like.

However, if desired, the pharmaceutical compositions of the invention may be in drink solution form upon dilution with water or any other aqueous system, to provide emulsion, e.g. microemulsion, and/or particulate systems suitable for drinking.

The pharmaceutical compositions of the invention are useful for the same indications as the poorly water soluble drugs. The pharmaceutical compositions are particularly useful for treatment and prevention of the conditions disclosed at pages 40 and 41 in EP 427 680, and at pages 5 and 6 in PCT/EP93/02604, the contents of which applications are incorporated herein by reference.

The pharmaceutical compositions comprising e.g. an immunosuppressant, e.g cyclosporin, as pharmacologicaly active agent, are particularly useful for:
a) treatment and prevention of organ or tissue transplant rejection, for example for the treatment of the recipients of heart, lung, combined heart-lung, liver, kidney, pancreatic, skin or corneal transplants. The pharmaceutical compositions are also indicated for the prevention of graft-versus-host disease, such as sometimes occurs following bone marrow transplantation;
b) treatment and prevention of autoimmune disease and of inflammatory conditions, in particular inflammatory conditions with an aetiology including an autoimmune component such as arthritis (for example rheumatoid arthritis, arthritis chronic progrediente and arthritis deformans) and rheumatic diseases; and
c) treatment of multi-drug resistance (MDR).

In a further aspect the present invention provides the use of a composition according to the present invention in the manufacture of a medicament for the treatment and prevention of an autoimmune or inflammatory condition or for the treatment and prevention of transplant rejection or for the treatment of multi-drug resistance.

The macrolide drugs also exhibit anti-tumour and antifungal activity and hence the pharmaceutical compositions can be used as anti-tumour and anti-fungal agents.

The contents of all the references referred to above especially the exemplified compounds are incorporated herein by reference, and each of the exemplified compounds may be used as a macrolide in the examples listed below.

### Examples

Following is a description by way of example only of compositions of this invention.
Unless otherwise indicated, components are shown in % by weight based on each composition.

### Solubilizing components:

Sunfat® GDC-N is from Taiyo Kagaku Co., Japan.

Oleyl alcohol from Henkel, Germany

Lauroglycol 90® from Gattefossé, France

Capryol 90® from Gattefossé, France

Sefsol 218® from Nikkol, Japan

### Surfactants:

Cremophor® RH 40 is from BASF, Germany.

SDS (Texapon K12®) from Fluka, Switzerland and Henkel, Germany

Solutol HS15® from BASF, Germany

Myrj 52® from Uniqema, Great Britain

Pluronic F68® from BASF, Germany

Brji 35® from Uniqema, Great Britain

Particle size measurements are made at 20°C at a dilution of 1 g composition in 10 to 100 ml water by photon correlation spectroscopy using, for example a Brookhaven BI-200 SM from Brookhaven Instruments, and by microscopy using, for example a Zeiss DMLB microscope.

### Example 1:

### Preparation of compositions

Compositions are made up by (i) dissolving the surfactant in water, (ii) dissolving the drug in the solubilizing component, and (iii) mixing the aqueous solution of the surfactant with the solution obtained by step (ii).

Particle size is measured by a Zetasizer or a microscope.

| COMPONENT | QUANTITY | | | | |
|---|---|---|---|---|---|
| | I | II | III | IV | |
| | % | % | % | % | |
| *(1) poorly soluble drug* | | | | | |
| Cyclosporin A | 7.7 | 12.5 | 9.7 | 7.9 | |

| *(2) solubilizing component* | | | | | |
|---|---|---|---|---|---|
| Oleyl alcohol | 30.8 | 29.2 | 22.6 | 18.4 | |

| *(3) surfactant* | | | | | |
|---|---|---|---|---|---|
| Sodium Lauryl Sulfate | 61.5 | 58.3 | 67.7 | 73.7 | |
| ratio composition:water (g) | 1.3+10 | 1.2+10 | 1.55+50 | 1.9+50 | |
| mean droplet size (nm)88.5 | 45.5 | 51.5 | 86.6 | | |

| | | | | | |
|---|---|---|---|---|---|
| | V | VI | VII | VIII | IX |
| | % | % | % | % | % |
| *(1) poorly soluble drug* | | | | | |
| Cyclosporin A | 2.7 | 2.7 | 2.7 | 18.2 | 18.2 |

| *(2) solubilizing component* | | | | | |
|---|---|---|---|---|---|
| Lauroglycol® 90 | 24.3 | - | - | - | - |
| Sefsol® 218 | - | 24.3 | - | - | - |
| Sunfat® GDC-N | - | - | 24.3 | - | 27.3 |
| Capryol® 90 | - | - | - | 27.3 | - |
| *(3) surfactant* | | | | | |
| Sodium Lauryl Sulfate | 73.0 | 73.0 | 73.0 | - | - |
| Cremophor® RH40 | - | - | - | 54.5 | 54.5 |
| ratio composition:water (g) | 1.85+10 | 1.85+10 | 1.85+10 | 1.1+50 | 1.1+50 |
| max droplet size (nm) | - | - | - | - | <12.5µm |
| | | | | | |

| | X | XI | XII | XIII | |
|---|---|---|---|---|---|
| | % | % | % | % | |
| *(1) poorly soluble drug* | | | | | |
| Cyclosporin A | 18.2 | 18.2 | 18.2 | 18.2 | |

| *(2) solubilizing component* | | | | | |
|---|---|---|---|---|---|
| Lauroglycol® 90 | 27.3 | - | - | - | |
| Capryol® 90 | - | - | 27.3 | - | |
| Sunfat® GDC-N | - | 27.3 | - | 27.3 | |
| *(3) surfactant* | | | | | |
| Solutol® HS15 | 54.5 | 54.5 | - | - | |
| Myrj® 52 | - | - | 54.5 | 54.5 | |
| ratio composition:water (g) | 1.1+50 | 1.1+50 | 1.1+50 | 1.1+50 | |
| max droplet size (µm) | 1.25 | <20 | <14 | <20 | |

| | | | | | |
|---|---|---|---|---|---|
| | XIV | XV | XVI | XVII | |
| | % | % | % | % | |
| *(1) poorly soluble drug* | | | | | |
| Cyclosporin A | 24.0 | 18.2 | 18.2 | 18.2 | |

| *(2) solubilizing component* | | | | | |
|---|---|---|---|---|---|
| Lauroglycol® 90 | - | 27.3 | 27.3 | - | |
| Capryol® 90 | 36.0 | - | - | - | |
| Sunfat® GDC-N | - | - | - | 27.3 | |

| *(3) surfactant* | | | | | |
|---|---|---|---|---|---|
| Pluronic® F68 | 40.0 | 54.5 | - | - | |
| Brij® 35 | - | - | 54.5 | 54.5 | |
| ratio composition:water (g) | 0.1+10 | 1.1+50 | 1.1+50 | 1.1+50 | |
| max droplet size (µm) | <7µm | <1.25 | <45 | <70 | |
| | | | | | |

| | XVIII | XIX | XX | XXI | |
|---|---|---|---|---|---|
| | % | % | % | % | |
| *(1) poorly soluble drug* | | | | | |
| Cyclosporin A | 25.0 | 25.0 | 18.2 | 14.3 | |

| *(2) solubilizing component* | | | | | |
|---|---|---|---|---|---|
| Lauroglycol® 90 | - | 37.5 | 27.3 | 21.4 | |
| Capryol® 90 | 37.5 | - | - | - | |

| *(3) surfactant* | | | | | |
|---|---|---|---|---|---|
| Sodium Lauryl Sulfate | 37.5 | 37.5 | 54.5 | 64.3 | |
| ratio composition:water (g) | 1.4+50 | 1.4+50 | 1.1+50 | 0.8+50 | |
| max droplet size (µm) | <7.5 | - | - | - | |

| | | | | | |
|---|---|---|---|---|---|
| | XXII | XXIII | XXIV | XXV | |
| | % | % | % | % | |
| *(1) poorly soluble drug* | | | | | |
| Cyclosporin A | 32.0 | 17.6 | 18.75 | 20.0 | |

| *(2) solubilizing component* | | | | | |
|---|---|---|---|---|---|
| Lauroglycol® 90 | - | 41.2 | 43.75 | 46.7 | |
| Capryol® 90 | 48.0 | - | - | - | |

| *(3) surfactant* | | | | | |
|---|---|---|---|---|---|
| Sodium Lauryl Sulfate | 20.0 | 41.2 | 37.5 | 33.3 | |
| ratio composition:water (g) | 0.1+10 | 1.7+10 | 0.16+10 | 0.15+10 | |
| mean droplet size (nm)20µm¹⁾ | 97.5 | 121.1 | 129.5 | | |

| | | | | | |
|---|---|---|---|---|---|
| ¹⁾max droplet size | | | | | |

The compositions are spray-dried together with Glucidex® as a carrier in an amount of about 23 % by weight of the total composition consisting of drug, solubilizing component, surfactant and carrier, and encapsulated into hard gelatine capsules or compressed to tablets.
Further examples may be made replacing Cyclosporin A by any of the drugs specified above, e.g. 2 mg 40-O-(2-hydroxy)ethyl-rapamycin, or 30 mg 33-epi-chloro-33-desoxy-ascomycin.

Other examples may be made by replacing Oleyl alcohol, Lauroglycol®90, Capryol®90, Sefsol®218, Sunfat®GDC-N by any of the solubilizing components specified above.

Other examples may be made by replacing Sodium lauryl sulfate, Cremophor®RH40, Solutol®HS15, Myrj®52, Pluronic®F68, Brij®35 by any of the surfactants specified above.

The examples illustrate compositions useful for example in the prevention of transplant rejection or for the treatment of autoimmune disease, on administration of from 1 to 5 unit dosages/day at a dose of 2 to 5 mg/kg per day. The examples are described with particular reference to Cyclosporin A but equivalent compositions may be obtained employing any macrolide or other drug.

On visual inspection after dilution, each of the compositions forms a stable microemulsion or emulsion.

## Claims

1. A pharmaceutical composition in solid form comprising
(i) a cyclosporin,
(ii) a solubilizing component,
(iii) a surfactant which is semisolid or solid at room temperature,
wherein the ratio of (iii) to (ii) is from 0.3 - 4 to 1, and which on dilution with an aqueous medium forms an emulsion or a microemulsion and/or a particulate system.

2. A composition according to claim 1 further comprising a carrier.

3. A composition according to claim 2 wherein the carrier is maltodextrin, lactose, gummi arabicum or gelatine.

4. A composition according to any preceding claim wherein the drug is Cyclosporin A.

5. A composition according to any preceding claim wherein the surfactant is polyethoxylated hydrogenated castor oil, polyoxyethylene fatty acid ester, polyoxyethylene-polyoxypropylene co-polymer, polyoxyethylene alkyl ether, or sodium lauryl sulfate.

6. A composition according to any preceding claim wherein the solubilizing component is a glyceryl mono- or di fatty acid ester, a propylene glycol mono- or di- fatty acid ester, or a fatty alcohol.

7. A composition according to any preceding claim wherein the drug dissolved in the solubilizing component is encapsulated in a polymeric matrix.

8. A composition according to any preceding claim in spray-dried form.

9. A process for the production of a composition according to claim 7 or 8 which process comprises
(i) dissolving the drug in the solubilizing component,
(ii) encapsulating the solution obtained by step (i) in a polymeric matrix,
(iii) spray drying or freeze drying the microparticles, optionally together with a carrier, to obtain a powder,
(iv) admixing the powder obtained by step (iii) with the surfactant.

10. A process for the production of a composition according to claim 8 which process comprises
(i) dissolving the surfactant in an aqueous solution,
(ii) dissolving the drug in the solubilizing component,
(iii) mixing the solution obtained by step (i) with the solution obtained by step (ii), and
(iv) spray-drying the mixture together with a carrier.

11. A composition obtainable by a process as claimed in claim 9 or 10 further worked up in the form of tablets or capsules.

12. A composition according to any one of claims 1 to 8 in form of a oral solid dosage form.

13. Use of a composition as claimed in any one of claims 1 to 8, 11 or 12 in the manufacture of a medicament for the treatment of autoimmune diseases or for the use as an immunosuppressant.
